# FASCICULE DE BREVET EUROPEEN

(11) **EP 3 727 409 B1**
(45) Date de publication et mention de la délivrance du brevet: **15.10.2025**
(21) Numéro de dépôt: 18833430.4
(22) Date de dépôt: 21.12.2018
(51) Int. Cl.: A61K 35/742, A61K 35/744, A23K 50/75, A23K 10/18, A61P 31/00

(54) **COMPOSITION COMPRENANT UN MÉLANGE DE BACILLUS SUBTILIS ET DE BACTÉRIE LACTIQUE DESTINEE A L'AMELIORATION DE LA SANTE DES JEUNES OVIPARES**
ZUSAMMENSETZUNG ENTHALTEND BACILLUS SUBTILIS UND MILCHSÄUREBAKTERIEN ZUR VERBESSUNG DER GESUNDHEIT VON EIERLEGENDEN TIEREN
COMPOSITION COMPRISING A COMBINATION OF BACILLUS SUBTILIS AND LACTIC ACID BACTERIA FOR IMPROVING HEALTH OF OVIPAROUS ANIMALS

(30) Priorité: 22.12.2017 FR 1763192
(43) Date de publication de la demande: 28.10.2020
(73) Titulaire: MIXSCIENCE, 35170 Bruz (FR)
(72) Inventeur: LAYUS, Michel, 22560 Pleumeur Bodou (FR); BRAME, Alexandre, 35000 Rennes (FR)
(74) Mandataire: LLR
(86) Numéro de dépôt international: PCT/EP2018/086643
(87) Numéro de publication internationale: WO 2019/122356

(56) Documents cités:
- WO-A1-2013/178947
- WO-A1-2019/092134
- YOUNES ABDELGAYED M ET AL: "Isolation and Pathogenicity Determination of Bacillus cereus Associated with Ulcer Formation in African Catfish Clarias gariepinus Phycoremediation: key issues for cost-effective nutrient removal processes. View project Nanotechnology View project", ARTICLEINASIAN JOURNAL OF ANIMAL SCIENCES, 1 November 2020 (2020-11-01), XP093046541, Retrieved from the Internet <URL:https://www.researchgate.net/publication/346967010_Isolation_and_Pathogenicity_Determination_of_Bacillus_cereus_Associated_with_Ulcer_Formation_in_African_Catfish_Clarias_gariepinus> [retrieved on 20230512]
- ZONGHUI ZUO: "Feed-borne Bacillus cereus exacerbates respiratory distress in chickens infected with Chlamydia psittaci by inducing haemorrhagic pneumonia", AVIAN PATHOLOGY, vol. 49, no. 3, 12 March 2020 (2020-03-12), GB, pages 251 - 260, XP093158785, ISSN: 0307-9457, DOI: 10.1080/03079457.2020.1716940
- GIARMA ELENI ET AL: "Defense systems in developing Artemia franciscana nauplii and their modulation by probiotic bacteria offer protection against a Vibrio anguillarum challenge", FISH & SHELLFISH IMMUNOLOGY, vol. 66, July 2017 (2017-07-01), pages 163 - 172, XP085057571
- MOHAPATRA SIPRA ET AL: "Dietary Multispecies Probiotic Supplementation Enhances the Immunohematological Responses and Reduces Mortality by Aeromonas hydrophila in Labeo rohita Fingerlings", JOURNAL OF THE WORLD AQUACULTURE SOCIETY, vol. 45, no. 5, October 2014 (2014-10-01), pages 532 - 544, XP002785569

## Description

L'invention concerne une composition destinée à l'amélioration de la santé des ovipares, et notamment des jeunes ovipares.

L'intérieur des œufs d'ovipares est stérile si l'intégrité de l'œuf est conservée. Lors de l'éclosion, le premier microbisme en contact avec le jeune ovipare sera donc celui présent sur l'œuf. Ce premier contact avec des microbes correspond à la première étape du processus de formation de l'écosystème microbien des ovipares qui se poursuivra par le contact avec l'écosystème de l'environnement de vie et de l'aliment des juvéniles.

Cependant, une contamination par un microorganisme pathogène est donc possible dès l'éclosion. Lors des premiers instants de vie, le microbisme des jeunes ovipares et leur système immunitaire étant en formation, ces derniers sont très vulnérables aux pathogènes.

Le biocontrôle des écosystèmes microbiens de l'extérieur de l'œuf, de l'environnement des juvéniles ovipares et de l'aliment apparaît donc primordial pour prévenir les risques de contamination de ces animaux et pour favoriser la bonne formation de leur microbisme.

Les décideurs demandent aux producteurs d'aliments pour animaux, aux agriculteurs, aux vétérinaires et aux organismes de réglementation de travailler ensemble pour déterminer les meilleures pratiques d'élevage et d'hygiène et des alternatives viables, afin de réduire l'utilisation d'antibiotiques chez les animaux d'élevage et d'améliorer le bien-être des animaux.

Pour répondre aux nouvelles exigences de l'agriculture moderne, et tenant compte des contraintes biologiques des ovipares, il est nécessaire de mettre au point de nouvelles méthodes de prévention de la contamination des jeunes animaux.

Les méthodes connues de l'état de la technique visent notamment à traiter l'environnement d'élevage des jeunes ovipares afin de favoriser leur croissance et leur bien-être. Par exemple, la demande WO2017132230 décrit une pulvérisation de probiotiques uniquement sur les jeunes poussins après éclosion afin d'augmenter les performances zootechniques en élevage des volailles.

D'autres méthodes connues de l'état de la technique visent quant à elles à influencer les performances via l'alimentation du jeune ovipare. On connait, par exemple, la demande EP1472933 A1 qui décrit une composition alimentaire destinée à améliorer la santé des poissons. La demande internationale WO9854981 vise quant à elle une supplémentation en flore bactérienne au stade précoce du développement des volailles.

Cependant, ces méthodes ne prennent pas en compte le biocontrôle du microbisme des œufs pourtant primordial dans le processus de formation du microbiote du jeune ovipare.

Aussi, l'invention a pour but de pallier cet inconvénient.

Un objet de l'invention est de proposer une méthode de prévention des risques sanitaires et d'amélioration de la performance des animaux ovipares d'élevage, ainsi qu'une composition microbienne.

L'invention concerne donc une composition comprenant ou consistant essentiellement en un mélange :
a. d'au moins une souche de bactérie du genre Bacillus qui est une souche de *Bacillus subtilis,* et
b. d'au moins une souche de bactérie lactique, ladite souche de bactérie lactique étant la souche de *Lactococcus lactis spp lactis* 1 souche NOL11, ladite souche étant déposée à la CNCM sous le numéro CNCM I - 4609.

pour son utilisation dans le cadre de la prévention des infections ou l'amélioration des performances zootechniques, notamment dans les premiers jours suivant l'éclosion, des animaux ovipares d'élevage,
ladite composition étant mise en contact direct avec les œufs desdits animaux ovipares d'élevage.

L'invention repose sur la constatation surprenante faite par les inventeurs qu'une application d'une composition bactérienne comprenant des souches de *Bacillus* et de bactéries lactiques, directement appliquée sur les œufs d'ovipares permet d'améliorer sensiblement la santé des nouveau-nés des ovipares, et de les protéger, dès le plus jeune âge (dès l'éclosion), contre les infections néfastes à leur développement, et ainsi favoriser et améliorer leur croissance.

La composition telle qu'utilisée dans l'invention présente l'avantage d'avoir une action interne à l'animal et dans son environnement extérieur, permettant de créer une flore de barrière dès le plus jeune âge.

Par « œuf », on entend dans l'invention une cellule vivante, environnée de réserves alimentaires et d'enveloppes protectrices, qui donnera un jeune d'une espèce considérée.

Dans l'invention, on entend par biocontrôle le fait d'orienter une population en favorisant et/ou limitant le développement de certaines espèces microbiennes dans un écosystème microbien donné, en utilisant des microorganismes ou des substances naturelles. On parle par exemple de biocontrôle de l'écosystème microbien du milieu d'élevage, de biocontrôle de l'écosystème microbien de l'aliment ou encore de biocontrôle de l'écosystème microbien intestinal.

Les ovipares selon l'invention sont des animaux qui pondent des œufs fécondés ou non, dont l'éclosion s'effectue hors du corps de l'animal, c'est-à-dire des animaux dont tout ou partie du développement embryonnaire, et éventuellement y compris la fécondation, est réalisé à l'extérieur du corps de l'animal.

Par « performances zootechniques » on entend dans l'invention les indicateurs chiffrés d'évaluation de l'aptitude biologique de l'animal pour la production pour laquelle il est élevé. Ces indicateurs étant notamment les performances pondérales (poids, gain moyen quotidien, indice de consommation...), les performances de reproduction (fertilité, fécondité, prolificité, taux d'éclosion...) ou les performances de production à proprement dit (production de viande, taux de ponte, mortalité...).

Par « les premiers jours suivant l'éclosion » on définit dans l'invention l'éclosion et les jours suivants, notamment les 5 jours suivant la sortie de l'œuf.

Pour les espèces ayant une durée de vie en élevage de moins d'un an, les premiers jours suivant l'éclosion peuvent s'étendre jusqu'à 30 jours. Pour les espèces ayant une durée de vie en élevage de plus d'un an, ce délai peut s'étendre jusqu'à 2 mois.

Plus précisément, les premiers jours suivant l'éclosion incluent le moment où le nouveau-né sort de l'œuf au sein duquel son développement embryonnaire s'est déroulé.

La composition selon l'invention est utilisée en contact direct avec l'œuf d'ovipares d'élevage, c'est-à-dire que la composition selon l'invention est appliquée soit sur les œufs par pulvérisation, incubation, trempage, étalement ou saupoudrage, soit dans le milieu d'incubation des œufs des ovipares (substrat ou surfaces des incubateurs, couveuses, écloserie et tous autres matériels d'élevage (notamment bacs, chariots, tiroirs, clayettes, grilles, ...en contact avec les oeufs avant éclosion). L'application de la composition selon l'invention directement sur les œufs permet le recouvrement des œufs par la solution et ainsi d'orienter et/ou contrôler la flore bactérienne présente à la surface des oeufs

L'objectif est que la composition selon l'invention soit sur l'œuf ou en contact avec l'œuf.

Dans l'invention, les œufs qui sont recouverts de la composition susmentionnée sont des oeufs placés en couveuse ou incubateur (ou en écloserie aquacole : réservoir). La couveuse, l'incubateur ou l'écloserie est l'endroit où sont placés les oeufs pour les maintenir à des températures idéales pour le développement de l'embryon. Ces oeufs peuvent notamment être isolés des animaux adultes.

Il est particulièrement avantageux dans l'invention que la composition susmentionnée comprenne au moins deux souches de Bacillus, et notamment au moins deux souches différentes de Bacillus et au moins une souche de bactérie lactique, ladite souche de bactérie lactique étant la souche de *Lactococcus lactis spp lactis* 1 souche NOL11, ladite souche étant déposée à la CNCM sous le numéro CNCM I - 4609, notamment de *Lactococcus lactis.*

Il est encore plus avantageux dans l'invention que la composition susmentionnée comprenne au moins trois souches de Bacillus, et notamment au moins trois souches différentes de Bacillus, et au moins une souche de bactérie lactique, notamment de *Lactococcus lactis.*

Il est encore plus avantageux dans l'invention que la composition susmentionnée comprenne au moins quatre souches de Bacillus, et notamment au moins quatre souches différentes de Bacillus et au moins une souche de bactérie lactique, notamment de *Lactococcus lactis.*

Outre l'amélioration des performances zootechniques, la composition selon l'invention est utilisée dans le cadre de la prévention des infections des ovipares d'élevage. Les infections desdits ovipares d'élevage sont notamment les infections dues à la prolifération de bactéries ou de virus pathogènes tels que : *Aeromonas salmonicida, Edwarsiella tarda, Flavobacterium spp Photobacterium damsela damsela, Stretococcus iniae, Streptococcus dysgalactiae, Streptococcus agalactiae, Tenacibaculum discolor, Tenacibaculum maritimum, Vibrio alginolyticus, Vibrio anguillarum, Vibrio harveyi, Vibrio parahaemolyticus, Yersinia ruckeri, Virus WSSv, Francisella noatunensis, Campylobacter jejuni, Chlamydia psittaci, Clostridium botulinum, Clostridium perfringeus, Escherichia coli, Erysipelothrix rhusiopathiae, Haemophilus paragallinarum, Listeria monocytogenes, Mycobacterium avium, Mycoplasma gallisepticum, Mycoplasma synoviae, Pasteurella multicoda, Pseudomonas aeruginosa, Riemerella anatipestier, Salmonella enterica, Staphylococcus aureus, Staphylococcus hyicus, Streptococcus bovis, Yersinia pseudotuberculosis, Salmonella spp,* et *Brachyspira spp.*

Avantageusement, l'invention concerne la composition susmentionnée pour son utilisation susmentionnée, dans laquelle la composition bactérienne comprend :
- au moins l'une des trois souches de *Bacillus subtilis* suivantes : NOL01, NOL02, NOL03, lesdites souches étant déposées à la CNCM sous les numéros respectifs : CNCM I - 4606, CNCM I-5043 et CNCM I - 4607, et
- au moins la souche de bactérie lactique *Lactococcus lactis spp lactis* 1 souche NOL11, ladite souche étant déposée à la CNCM sous le numéro CNCM I - 4609.

L'invention concerne l'utilisation d'au moins une souche choisie parmi les souches NOL01, NOL02 et NOL03 et au moins la souche NOL11. Aussi, l'invention couvre les 7 combinaisons suivantes :
- NOL01 et NOL11,
- NOL02 et NOL11,
- NOL03 et NOL11,
- NOL01, NOL02 et NOL11,
- NOL01, NOL03 et NOL11,
- NOL02, NOL03 et NOL11, et
- NOL01, NOL02, NOL03 et NOL11.

Toutes ces souches ont été déposées à la collection nationale des microorganismes (CNCM) à l'Institut Pasteur à Paris, selon le traité de Budapest.

Avantageusement, l'invention concerne la composition susmentionnée pour son utilisation susmentionnée, ladite composition comprenant de 10⁴ à 10¹¹ colonies bactériennes de Bacillus et de 10⁴ à 10¹¹ colonies bactériennes de bactérie lactique, les colonies bactériennes étant par mL ou g de composition.

Par souche de bactéries, on entend dans l'invention l'ensemble des individus (bactéries) issus de repiquages successifs d'une colonie bactérienne. De plus dans l'invention, une colonie bactérienne correspond à une unité formant colonie (UFC ou CFU en anglais).

En d'autres termes, dans ce mode de réalisation avantageux, si la composition selon l'invention est sous forme liquide, ladite composition comprendra de 10⁴ à 10¹¹ colonies bactériennes de *Bacillus* par mL de composition, et cela pour chacune des souches lorsque la composition comprend au moins deux souches, et de 10⁴ à 10¹¹ colonies bactériennes de bactérie lactique par mL de composition.

Si par contre la composition selon l'invention est déshydratée ou non aqueuse, ladite composition comprendra de 10⁴ à 10¹¹ colonies bactériennes de *Bacillus* par g de composition, et cela pour chacune des souches lorsque la composition comprend au moins deux souches, et de 10⁴ à 10¹¹ colonies bactériennes de bactérie lactique par g de composition.

Dans l'invention, de 10⁴ à 10¹¹ colonies bactériennes signifie : environ 10⁴, environ 5. 10⁴, environ 10⁵, environ 5.10⁵, environ 10⁶, environ 5.10⁶, environ 10⁷, environ 5.10⁷, environ 10⁸, environ 5.10⁸, environ 10⁹, environ 5.10⁹, environ 10¹⁰, environ 5.10¹⁰ ou environ 10¹¹ colonies bactériennes.

L'homme du métier sait aisément comment déterminer ce nombre de bactéries, notamment par comptage soit manuel (en utilisant une lame de Malassez) ou en utilisant un compteur de cellule automatique, ou par dilution puis ensemencement sur gélose et comptage des colonies.

Encore plus avantageusement, l'invention concerne la composition susmentionnée pour son utilisation susmentionnée, où les souches de *Bacillus* sont sous forme sporulée et/ou sous forme végétative.

Les bactéries lactiques sont quant à elles toujours sous forme végétative. Aussi la composition selon l'invention comprend au moins une souche de *Bacillus* sous forme sporulée et au moins une souche de bactérie lactique sous forme végétative, ou comprend au moins une souche de *Bacillus* sous forme végétative et au moins une souche de bactérie lactique sous forme végétative, ladite souche de bactérie lactique étant la souche de *Lactococcus lactis spp lactis* 1 souche NOL11, ladite souche étant déposée à la CNCM sous le numéro CNCM I - 4609

De manière avantageuse, l'invention concerne la composition susmentionnée pour son utilisation susmentionnée, où ladite composition est mise en contact avec lesdits œufs par pulvérisation, incubation, trempage, étalement ou saupoudrage.

L'homme du métier, selon le mode d'application utilisé, saura choisir le matériel le plus approprié, et notamment préfèrera une composition liquide pour la pulvérisation, le trempage ou l'étalement et favorisera une composition déshydratée pour une application par saupoudrage.

Avantageusement, l'invention concerne la composition susmentionnée pour son utilisation susmentionnée, ladite composition étant en outre en contact avec les nouveau-nés ayant éclos desdits œufs desdits animaux ovipares d'élevage et/ou en contact avec l'alimentation fournie aux nouveau-nés ayant éclos desdits œufs desdits animaux ovipares d'élevage.

Avantageusement, l'invention concerne la composition susmentionnée pour son utilisation susmentionnée, ladite composition étant en outre épandue dans l'environnement où sont placés lesdits œufs et lesdits nouveau-nés ayant éclos desdits oeufs desdits animaux ovipares d'élevage.

L'application dans l'environnement permet de contrôler et/ou d'orienter la flore bactérienne de l'environnement des animaux d'élevage terrestre en diminuant la pression pathogène. Elle peut se faire en nébulisation ou pulvérisation des bâtiments d'élevage, incorporation à la litière... On observe moins de contamination des animaux car il y a moins de foyers pathogènes. Une telle application joue sur les pathogènes vivants ou survivants dans l'environnement. Les modes d'épandage dans l'environnement sont la pulvérisation, la nébulisation, le recouvrement des surfaces par des mousses, le trempage, la balnéation ou encore le saupoudrage de poudre.

L'épandage se fait généralement sur les surfaces de l'élevage, principalement sur les surfaces en contact direct ou potentiel avec les animaux, de préférence sur des surfaces nettoyées et désinfectées, après avoir attendu la fin de la rémanence des produits désinfectants.

L'épandage dans l'environnement est réalisé au moins une fois après un vide sanitaire, après un protocole de nettoyage / désinfection ou avant arrivée des animaux. Il est particulièrement avantageux de pratiquer une application répétée toutes les semaines.

L'application sur les nouveau-nés et/ou dans la nourriture peut être combinée à l'application dans l'environnement.

L'application sur les nouveau-nés permet de contrôler ou d'orienter le microbisme de l'animal en diminuant la pression pathogène. L'application peut se faire en nébulisation, pulvérisation, trempage, brumisation, pédiluve, mousse, balnéation, selon la zone de l'animal sur laquelle il est souhaité de déposer la composition selon l'invention. Cela génère moins de contamination des animaux car il y a moins de foyers pathogènes directement sur l'animal.

Il est avantageux d'appliquer au moins une fois la composition sur l'animal le plus tôt possible dans la vie de l'animal. De préférence l'application est répétée toutes les semaines.

De manière avantageuse, on utilisera de 10³ à 10⁹ CFU par oeuf ou nouveau-né pour une application chez les espèces de poissons ou crustacés et de 10⁴ à 10¹⁰ CFU par oeuf ou nouveau-né pour une application chez les volailles.

Dans l'invention, de 10³ à 10⁹ CFU signifie : environ 10³, environ 5.10³, environ 10⁴, environ 5.10⁴, environ 10⁵, environ 5.10⁵, environ 10⁶, environ 5.10⁶, environ 10⁷, environ 5.10⁷, environ 10⁸, environ 5.10⁸, environ 10⁹ CFU. Il est également possible de fournir la composition selon l'invention dans l'alimentation des nouveau-nés.

Encore plus avantageusement, l'invention concerne une composition pour son utilisation susmentionnée, ladite composition consistant essentiellement en
- les trois souches de *Bacillus subtilis* suivantes : NOL01, NOL02, NOL03, lesdites souches étant déposées à la CNCM sous les numéros respectifs : CNCM I - 4606, CNCM I-5043 et CNCM I - 4607, et
- la souche de bactérie lactique *Lactococcus lactis spp lactis* 1 souche NOL11, ladite souche étant déposée à la CNCM sous le numéro CNCM I - 4609.

Cette combinaison est particulièrement avantageuse et décrite dans les exemples.

Il est particulièrement avantageux que la composition comprenne les trois souches de bactéries *Bacillus subtilis* susmentionnées, sous forme végétative et / ou sporulée, et la souche de bactérie lactique susmentionnée, sous forme végétative

Avantageusement, l'invention concerne la composition susmentionnée pour son utilisation susmentionnée, où lesdits animaux ovipares d'élevages sont des oiseaux, des reptiles, et ladite composition est appliquée sur la coquille des œufs desdits oiseaux ou desdits reptiles.

Les oiseaux particulièrement avantageux concernés dans le cadre de l'invention sont les oiseaux d'élevages tels que les gallinacés, notamment les dindes, les poules, les pintades, les cailles et les faisans, mais également les canards, les autruches, les pigeons, les perdrix et les oies.

Les reptiles envisagés dans l'invention sont les tortues terrestres, certains serpents et les crocodiliens.

Dans le cadre des oiseaux susmentionnés, l'œuf est recouvert par la composition selon l'invention. Ce recouvrement peut être total ou partiel.

Avantageusement, l'invention concerne la composition susmentionnée pour son utilisation susmentionnée, où lesdits animaux ovipares d'élevages sont des ovipares aquatiques et ladite composition est dispersée dans l'eau des bassins d'élevage desdits ovipares aquatiques afin d'être mise en contact avec lesdits œufs, ou afin d'être mise directement en contact avec lesdits œufs.

Dans ce mode de réalisation avantageux, les ovipares sont des ovipares aquatiques tels que des poissons d'élevage, les salamandres, les tritons, les crapauds les grenouilles, les crustacés, notamment les crevettes, les reptiles aquatiques, tels que certains serpents, et les tortues d'eau.

Dans la mesure où les œufs des animaux ovipares aquatiques sont dans l'eau, la composition selon l'invention qui est appliquée sur les œufs est notamment dispersée dans l'eau des bassins contenant lesdits œufs. Comme mentionné précédemment, l'essentiel est que l'œuf soit en contact avec la composition selon l'invention, de sorte que le nouveau-né soit immédiatement en contact avec la flore lors de la sortie de l'œuf.

Dans l'invention, on définit par bassin d'élevage, toute structure utilisée au sein d'un élevage aquacole contenant les œufs et les nouveau-nés.

Avantageusement, l'invention concerne la composition susmentionnée pour son utilisation susmentionnée, où ladite au moins une souche de bactérie du genre Bacillus et ladite au moins une souche de bactérie lactique sont utilisées simultanément, séparément ou de manière étalée dans le temps.

Dans la cadre d'une application séparée, il est par exemple possible de fournir dans un bassin, les Bacillus selon l'invention à une extrémité du bassin, et la bactérie lactique à une autre extrémité du bassin.

Dans la cadre d'une application étalée dans le temps, il est possible d'appliquer d'abord soit les Bacillus soit la bactérie lactique, puis après une période de temps déterminée, d'appliquer l'autre type de bactéries.

L'invention sera mieux comprise à la lumière des exemples ci-dessous, et des figures ci-après décrites.

### Légende des figures

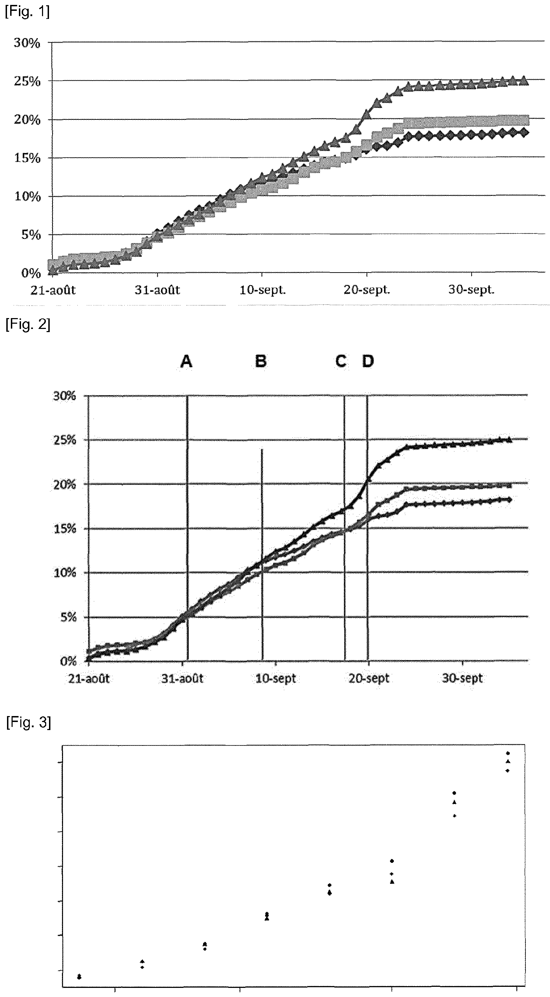

[Fig. 1]
   Le graphique de la **figure** 1 représente la mortalité cumulée des alevins après éclosion dans les auges de deux essais (losanges et carrés) et dans l'auge témoin (triangles). Cette mortalité cumulée est exprimée en pourcentage du nombre d'œufs éclos en fonction du temps (en jours).
[Fig. 2]
   Le graphique de la **figure** 2 représente le même graphique que la figure 1 sur lequel les événements pathologiques ont été mentionnés. A :Costiase très sévère, B : Flavobactériose septicémique/costiase très sévère, C : Costiase sévère et D : Costiase très sévère.
[Fig. 3]
   **La** **figure** 3 représente le poids moyen des alevins dans les différents lots. L'axe des abscisses représente le temps en jours et celui des ordonnées le poids moyen des alevins en grammes. Les points en forme de losanges représentent les poids moyens des alevins appartenant au lot témoin, les triangles ceux du lot ayant reçu un trempage des œufs suivi d'une application de la composition dans l'environnement et les ronds ceux du lot ayant reçu un trempage des œufs suivi d'une application de la composition dans l'aliment.
[Fig. 4]

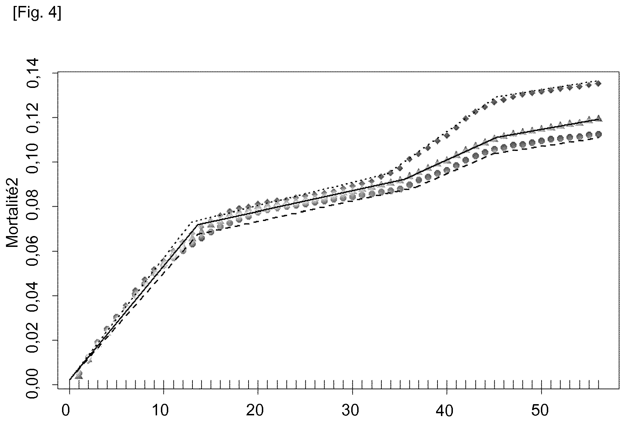

   La **figure 4** représente la mortalité cumulée des alevins au cours du temps dans les différents lots. L'axe des abscisses représente le temps en jours et celui des ordonnées le pourcentage de mortalité. Les points en forme de losanges représentent la mortalité dans le lot témoin, les triangles la mortalité dans le lot ayant reçu un trempage des œufs suivi d'une application de la composition dans l'environnement et les ronds la mortalité dans le lot ayant reçu un trempage des œufs suivi d'une application de la composition dans l'aliment. Les courbes représentent les modèles statistiques associés aux mortalités réelles avec en pointillés fins le modèle du lot témoin, en trait plein le modèle du lot application trempage des œufs et environnement et enfin les pointillés épais le lot application trempage des œufs et aliment.

### EXEMPLES

### Exemple 1 - efficacité de l'application de la composition par trempage des oeufs et dans l'environnement sur les alevins de truite arc en ciel

L'objectif de cet essai est de déterminer l'efficacité de la composition selon l'invention sur les alevins de truite arc en ciel. L'application de la composition étant réalisé sur les œufs et dans l'environnement des animaux.

### Protocole :

L'expérience est réalisée dans un élevage sur une bande de production.

La bande correspond à 3 auges d'éclosion avec 60 000 œufs par auge. Une servira de témoin et les deux autres recevront les applications de la composition.

Les deux types d'application sont réalisés :
- Trempage des œufs :
   Les œufs sont trempés pendant 4 minutes dans 2L d'eau de rivière pour le lot témoin et dans 2L d'eau de rivière mélangés avec une demi-dose de produit pour le lot essai.

Une dose de composition selon l'invention a été préparée à partir de deux flacons distincts de 20 mL :
- Le flacon 1 contenant les Bacillus NOL01, NOL02 et NOL03, à une concentration d'environ 10⁹ CFU/mL des 3 Bacillus,
- Le flacon 2 contenant les Lactococcus NOL11, à une concentration d'environ 10⁹ CFU/mL.

### • Pulvérisation sur l'environnement :

Les auges et clayettes des lots essais sont traitées avec 2L d'eau de rivière mélangés à une demi-dose de produit chacune avant mise en eau.

Ce traitement a été répété juste avant l'éclosion, en fin d'éclosion, en fin de résorption vitelline puis une fois par semaine jusqu'à la date de transfert des alevins dans le parc de grossissement. Pour chacun de ces traitements, l'eau des 3 auges est abaissée au minimum pour ne pas nuire à la santé des alevins puis remontée à son niveau normal après le traitement des auges essais. La durée totale de l'essai est d'environ 2 mois, période entre l'arrivée des oeufs dans l'élevage et le transfert des alevins. Ce qui fait un total d'environ 10 traitements.

Les pulvérisations ont été réalisées à l'aide d'un pulvérisateur manuel (type pulvérisateur de jardin).

### Mesures et résultats

Lors de l'essai, la mortalité journalière dans les trois auges est relevée, les événements sanitaires sont également notés.

La **figure 1** montre que la mortalité cumulée semble à peu près équivalente entre les différentes auges jusqu'au 10 septembre où on observe une augmentation de la mortalité moins importante dans les auges essais. L'écart de mortalité entre les auges essais et l'auge témoin se creuse encore plus autour du 19 septembre. Au final, on observe 3000 morts de moins dans les auges essais que dans celle témoin au 21 août, soit une baisse de mortalité de 20 à 25%.

Ces deux dates correspondent à deux épisodes sanitaires que sont une flavobactériose septicémique et une costiase comme le montre la **figure 2****.**

L'eau détournée de la rivière arrivant au niveau des auges étant la même pour les 3 auges, la pression pathogène est également équivalente. Ces résultats montrent donc que l'application de la composition comme mentionnée ci-dessus permet de prévenir la mortalité des alevins lors d'un épisode sanitaire.

### Conclusion

L'application de la composition par trempage sur les oeufs suivie d'une application dans l'environnement des alevins de truite arc en ciel permet de limiter les impacts d'une pression pathogène sur la santé des alevins.

### Exemple 2 - efficacité de l'application de la composition par trempage des œufs et, dans l'environnement ou via l'aliment sur les alevins de truite arc en ciel

L'objectif de cet essai est de déterminer l'efficacité de la composition selon l'invention sur les alevins de truite arc en ciel. L'application de la composition étant réalisée sur les œufs et, dans l'environnement des animaux ou sur les œufs et via l'aliment.

### Protocole :

L'expérience est réalisée dans un élevage sur une bande de production.

La bande correspond à 3 auges d'éclosion avec 60 000 œufs par auge. L'une sert de témoin (auge 5) et les deux autres (auge 2 et auge 3) reçoivent les applications de la composition selon l'invention.

Les applications réalisées sont les suivantes :
- Trempage des oeufs :
   Les œufs ont été trempés pendant 5 minutes dans 2L d'eau de source pour le lot témoin et dans 2L d'eau de source mélangés avec une demi-dose de produit pour les lots essais, comme définie à l'exemple 1.
- Application dans l'environnement :
   Avant mise en eau des auges :
      Auge essai 3 : La veille de l'arrivée des œufs, l'auge est mise en eau avec une dose de flores puis mise en recirculation pendant 1 journée.
   Après mise en eau des auges :
      Auges essai 2, 3 et auge témoin 5 : avant chaque application, l'eau des auges est abaissée au minimum pour ne pas nuire à la santé des alevins puis remontée à son niveau normal après application des flores dans l'auge essai 3.
      Auge essai 3 : Les parois de l'auge émergées sont pulvérisées avec 1L d'eau de source mélangé à une demi-dose de produit.
      Ce traitement est répété juste avant l'éclosion, en fin d'éclosion, en fin de résorption vitelline puis une fois par semaine jusqu'à la date de transfert des alevins dans le parc de grossissement. Les pulvérisations ont été réalisées à l'aide d'un pulvérisateur manuel (type pulvérisateur de jardin).
   • Application dans l'aliment :
      Auge essai 2 : Une fois par semaine, dès le premier jour d'alimentation, une demi dose de flore sera mélangée à la main dans l'aliment avant d'alimenter les alevins. L'incorporation des flores à l'aliment se fera environ 1 heure avant la distribution. La demi-dose de flores sera mélangée dans un seau avec l'aliment avant distribution.

### Mesures et résultats

Lors de l'essai, on a relevé la mortalité journalière et le poids moyen hebdomadaire des alevins dans chaque auge ainsi que les événements sanitaires.

**[Table 1]**

| Poids moyen des alevins par auge (g) | | | | | | | | |
|---|---|---|---|---|---|---|---|---|
| | 27 j | 34 j | 41 j | 48 j | 55 j | 62 j | 69 j | 75 j |
| Auge 2 | 0.156 | 0.25 | 0.35 | 0.515 | 0.687 | 0.826 | 1.22 | 1.45 |
| Auge 3 | 0.161 | 0.25 | 0.34 | 0.495 | 0.644 | 0.704 | 1.16 | 1.4 |
| Auge 5 | 0.172 | 0.217 | 0.32 | 0.526 | 0.635 | 0.752 | 1.09 | 1.35 |

La **figure 3** montre que l'application de ladite composition par trempage des œufs et via l'aliment ou dans l'environnement améliore le poids moyen des alevins avec un gain en fin d'alevinage de respectivement 7,4% et 3,7% de poids supplémentaire aux alevins du lot témoin.

La **figure 4** montre que les deux lots avec l'application de ladite composition par trempage des œufs et via l'aliment ou dans l'environnement ont été moins impactés par l'épisode sanitaire survenu à environ 33 jours dans l'ensemble des auges. Ceci se traduit par une mortalité des alevins significativement moins importante (entre 12 et 16% de mortalité en moins par rapport au lot témoin).

### Conclusion

L'application de la composition selon l'invention par trempage sur les œufs suivie d'une application dans l'environnement ou dans l'alimentation des alevins de truite arc-en-ciel améliore le gain de poids des alevins et réduit la mortalité des animaux.

### Exemple 3 - application volaille.

Des expériences sur les œufs de volailles (poules) ont été réalisées.

L'invention n'est pas limitée aux modes de réalisation présentés et d'autres modes de réalisation apparaîtront clairement à l'homme du métier.

## Revendications

1. Composition comprenant ou consistant essentiellement en un mélange :
a. d'au moins une souche de bactérie du genre Bacillus, qui est une souche de *Bacillus subtilis,* et
b. d'au moins une souche de bactérie lactique, ladite souche de bactérie lactique étant la souche de *Lactococcus lactis spp lactis* 1 souche NOL11, ladite souche étant déposée à la CNCM sous le numéro CNCM I - 4609,
pour son utilisation pour la prévention des infections des animaux ovipares d'élevage,
ladite composition étant mise en contact direct avec les œufs desdits animaux ovipares d'élevage.

2. Composition pour son utilisation selon la revendication 1, dans laquelle la composition bactérienne comprend :
- au moins l'une des trois souches de *Bacillus subtilis* suivantes : NOL01, NOL02, NOL03, lesdites souches étant déposées à la CNCM sous les numéros respectifs : CNCM I - 4606, CNCM I-5043 et CNCM I - 4607, et
- au moins la souche de bactérie lactique: *Lactococcus lactis spp lactis* 1 souche NOL11, ladite souche étant déposée à la CNCM sous le numéro CNCM I - 4609.

3. Composition pour son utilisation selon la revendication 1 ou la revendication 2, ladite composition comprenant de 10⁴ à 10¹¹ colonies bactériennes de *Bacillus* et de 10⁴ à 10¹¹ colonies bactériennes de bactérie lactique, les colonies bactériennes étant par mL ou g de composition.

4. Composition pour son utilisation selon l'une quelconque des revendications 1 à 3, où les souches de *Bacillus* sont sous forme sporulée et/ou sous forme végétative.

5. Composition pour son utilisation selon l'une quelconque des revendications 1 à 4, où ladite composition est mise en contact avec lesdits œufs par pulvérisation, incubation, trempage, étalement ou saupoudrage.

6. Composition pour son utilisation selon l'une quelconque des revendications 1 à 5, ladite composition étant en outre en contact avec les nouveau-nés ayant éclos desdits œufs desdits animaux ovipares d'élevage et/ou en contact avec l'alimentation fournie aux nouveau-nés ayant éclos desdits œufs desdits animaux ovipares d'élevage.

7. Composition pour son utilisation selon l'une quelconque des revendications 1 à 6, ladite composition consistant essentiellement en
- les trois souches de *Bacillus subtilis* suivantes : NOL01, NOL02, NOL03, lesdites souches étant déposées à la CNCM sous les numéros respectifs : CNCM I - 4606, CNCM I-5043 et CNCM I - 4607, et
- la souche de bactérie lactique: *Lactococcus lactis spp lactis* 1 souche NOL11, ladite souche étant déposée à la CNCM sous le numéro CNCM I - 4609.

8. Composition pour son utilisation selon l'une quelconque des revendications 1 à 7, où lesdits animaux ovipares d'élevages sont des oiseaux et ladite composition est appliquée sur la coquille des œufs desdits oiseaux.

9. Composition pour son utilisation selon l'une quelconque des revendications 1 à 7, où lesdits animaux ovipares d'élevages sont des ovipares aquatiques et ladite composition est dispersée dans l'eau des bassins d'élevage desdits ovipares aquatiques et mise directement en contact avec lesdits œufs.

10. Composition pour son utilisation selon l'une quelconque des revendications 1 à 9, où lesdites infections des animaux ovipares d'élevage surviennent dans les premiers jours suivant l'éclosion.

11. Composition pour son utilisation selon l'une quelconque des revendications 1 à 10, où ladite au moins une souche de bactérie du genre Bacillus et ladite au moins une souche de bactérie lactique sont utilisées simultanément, séparément ou de manière étalée dans le temps.

12. Utilisation d'une composition comprenant ou consistant essentiellement en un mélange :
a. d'au moins une souche de bactérie du genre Bacillus qui est une souche de *Bacillus subtilis,* et
b. d'au moins une souche de bactérie lactique, ladite souche de bactérie lactique étant la souche de *Lactococcus lactis spp lactis* 1 souche NOL11, ladite souche étant déposée à la CNCM sous le numéro CNCM I - 4609,
pour l'amélioration des performances zootechniques des animaux ovipares d'élevage, ladite composition étant mise en contact direct avec les œufs desdits animaux ovipares d'élevage.

13. Utilisation selon la revendication 12, dans laquelle la composition comprend :
- au moins l'une des trois souches de *Bacillus subtilis* suivantes : NOL01, NOL02, NOL03, lesdites souches étant déposées à la CNCM sous les numéros respectifs : CNCM I - 4606, CNCM I-5043 et CNCM I - 4607, et
- au moins la souche de bactérie lactique: *Lactococcus lactis spp lactis* 1 souche NOL11, ladite souche étant déposée à la CNCM sous le numéro CNCM I - 4609.

14. Utilisation selon la revendication 12 ou 13, dans laquelle la composition comprend :
- les trois souches de *Bacillus subtilis* suivantes : NOL01, NOL02, NOL03, lesdites souches étant déposées à la CNCM sous les numéros respectifs : CNCM I - 4606, CNCM I-5043 et CNCM I - 4607, et
- la souche de bactérie lactique: *Lactococcus lactis spp lactis* 1 souche NOL11, ladite souche étant déposée à la CNCM sous le numéro CNCM I - 4609.

## Patentansprüche

1. Zusammensetzung, umfassend oder im Wesentlichen bestehend aus einer Mischung:
a. von mindestens einem Bakterienstamm der Gattung Bacillus, der ein Stamm von *Bacillus subtilis* ist, und
b. von mindestens einem Milchsäurebakterienstamm, wobei der Milchsäurebakterienstamm der Stamm von *Lactococcus lactis spp lactis 1* Stamm NOL11 ist, wobei der Stamm bei der CNCM unter der Nummer CNCM I - 4609 hinterlegt ist,
für ihre Verwendung für die Vorbeugung von Infektionen bei eierlegenden Nutztieren, wobei die Zusammensetzung in direkten Kontakt mit den Eiern der eierlegenden Nutztiere gebracht wird.

2. Zusammensetzung für ihre Verwendung nach Anspruch 1, wobei die Bakterienzusammensetzung umfasst:
- mindestens einen der folgenden drei Stämme von *Bacillus subtilis:* NOL01, NOL02, NOL03, wobei die Stämme bei der CNCM unter den jeweiligen Nummern hinterlegt sind: CNCM I - 4606, CNCM I-5043 und CNCM I - 4607, und
- mindestens den Milchsäurebakterienstamm: *Lactococcus lactis spp lactis* 1 Stamm NOL11, wobei der Stamm bei der CNCM unter der Nummer CNCM I - 4609 hinterlegt ist.

3. Zusammensetzung für ihre Verwendung nach Anspruch 1 oder 2, die Zusammensetzung umfassend von 10⁴ bis 10¹¹ Bakterienkolonien von *Bacillus* und von 10⁴ bis 10¹¹ Bakterienkolonien von Milchsäurebakterien, wobei die Bakterienkolonien pro ml oder g der Zusammensetzung sind.

4. Zusammensetzung für ihre Verwendung nach einem der Ansprüche 1 bis 3, wobei die Stämme von *Bacillus* in sporulierter Form und/oder in vegetativer Form sind.

5. Zusammensetzung für ihre Verwendung nach einem der Ansprüche 1 bis 4, wobei die Zusammensetzung durch Besprühen, Inkubieren, Eintauchen, Verteilen oder Bestäuben mit den Eiern in Kontakt gebracht wird.

6. Zusammensetzung für ihre Verwendung nach einem der Ansprüche 1 bis 5, wobei die Zusammensetzung ferner mit den Neugeborenen, die aus den Eiern der eierlegenden Nutztiere geschlüpft sind, und/oder mit dem Futter in Kontakt kommt, das den Neugeborenen bereitgestellt wird, die aus den Eiern der eierlegenden Nutztiere geschlüpft sind.

7. Zusammensetzung für ihre Verwendung nach einem der Ansprüche 1 bis 6, wobei die Zusammensetzung im Wesentlichen besteht aus
- den drei folgenden Stämmen von *Bacillus subtilis:* NOL01, NOL02, NOL03, wobei die Stämme bei der CNCM unter den jeweiligen Nummern hinterlegt sind: CNCM I - 4606, CNCM I-5043 und CNCM I - 4607, und
- dem Milchsäurebakterienstamm: *Lactococcus lactis spp lactis 1* Stamm NOL11, wobei der Stamm bei der CNCM unter der Nummer CNCM I - 4609 hinterlegt ist.

8. Zusammensetzung für ihre Verwendung nach einem der Ansprüche 1 bis 7, wobei die eierlegenden Nutztiere Vögel sind und die Zusammensetzung auf die Schale der Eier der Vögel aufgetragen wird.

9. Zusammensetzung für ihre Verwendung nach einem der Ansprüche 1 bis 7, wobei die eierlegenden Nutztiere im Wasser lebende eierlegende Tiere sind und die Zusammensetzung in dem Wasser der Zuchtbecken der im Wasser lebenden eierlegenden Tiere verstreut und direkt mit den Eiern in Kontakt gebracht wird.

10. Zusammensetzung für ihre Verwendung nach einem der Ansprüche 1 bis 9, wobei die Infektionen der eierlegenden Nutztiere innerhalb der ersten paar Tage nach dem Schlüpfen auftreten.

11. Zusammensetzung für ihre Verwendung nach einem der Ansprüche 1 bis 10, wobei der mindestens eine Stamm von Bakterien der Gattung Bacillus und der mindestens eine Milchsäurebakterienstamm gleichzeitig, getrennt oder zeitlich verteilt verwendet werden.

12. Verwendung einer Zusammensetzung, umfassend eine Mischung oder im Wesentlichen bestehend aus:
a. mindestens einem Bakterienstamm der Gattung Bacillus, der ein Stamm *von Bacillus subtilis* ist, und
b. mindestens einem Milchsäurebakterienstamm, wobei der Milchsäurebakterienstamm der Stamm von *Lactococcus lactis spp lactis 1* Stamm NOL11 ist, der bei der CNCM unter der Nummer CNCM I - 4609 hinterlegt ist,
für die Verbesserung der tierzüchterischen Leistungen von eierlegenden Nutztieren, wobei die Zusammensetzung in direkten Kontakt mit den Eiern der eierlegenden Nutztiere gebracht wird.

13. Verwendung nach Anspruch 12, wobei die Zusammensetzung umfasst:
- mindestens einen der folgenden drei Stämme von *Bacillus subtilis:* NOL01, NOL02, NOL03, wobei die Stämme bei der CNCM unter den jeweiligen Nummern hinterlegt sind:
5 CNCM I - 4606, CNCM I-5043 und CNCM I - 4607, und
- mindestens den Milchsäurebakterienstamm: *Lactococcus lactis spp lactis* 1 Stamm NOL11, wobei der Stamm bei der CNCM unter der Nummer CNCM I - 4609 hinterlegt ist.

14. Verwendung nach Anspruch 12 oder 13, wobei die Zusammensetzung umfasst:
- die drei folgenden Stämme von *Bacillus subtilis:* NOL01, NOL02, NOL03, wobei die Stämme bei der CNCM unter den jeweiligen Nummern hinterlegt sind: CNCM I - 4606, CNCM I-5043 und CNCM I - 4607, und
- den Milchsäurebakterienstamm: *La ctococcus lactis spp lactis 1* Stamm NOL11, wobei der Stamm bei der CNCM unter der Nummer CNCM I - 4609 hinterlegt ist.

## Claims

1. Composition comprising or consisting essentially of a mixture
a. at least one strain of a bacterium of the genus Bacillus, which is a strain of *Bacillus subtilis;* and
b. at least one strain of lactic acid bacterium, said strain of lactic acid bacterium being the *Lactotoccus lactis spp lactis 1* strain NOL11, said strain being deposited at CNCM under number CNCM I - 4609,
for the prevention of infections of farmed oviparous animals,
said composition being placed in direct contact with the eggs of said farmed oviparous animals.

2. Composition for its use according to claim 1, wherein the bacterial composition comprises:
- at least one of the following three strains of *Bacillus subtilis:* NOL01, NOL02, NOL03, said strains being deposited at the CNCM under respective numbers: CNCM I- 4606, CNCM 1-5043 and CNCM I - 4607, and
- at least the strain of lactic acid bacterium: *Lactococcus lactic spp lactis* 1 strain NOL11, said strain being deposited at the CNCM under number CNCM 1-4609.

3. Composition for its use according to claim 1 or claim 2, said composition comprising from 10⁴ to 10¹¹ bacterial colonies of Bacillus and from 10⁴ to 10¹¹ bacterial colonies of lactic acid bacterium, the bacterial colonies being per mL or g of composition.

4. Composition for its use according to anyone of claims 1 to 3, wherein the strains of Bacillus are in sporulated form and/or in vegetative form.

5. Composition for its use according to anyone of claims 1 to ', wherein said composition is placed in contact with said eggs by spraying, incubation, soaking, spreading or powder dusting.

6. Composition for its use according to anyone of claims 1 to 5, said composition further being in contact with the newborns having hatched from said eggs from said farmed oviparous animals and/or in contact with the food supplied to the newborns having hatched from said eggs from said farmed oviparous animals.

7. Composition for its use according to anyone of claims 1 to 6, said composition essentially consisting of
- the following three strains of *Bacillus subtilis:* NOL01, NOL02, NOL03, said strains being deposited at the CNCM under the respective numbers: CNCM I - 4606, CNCM 1-5043 and CNCM I - 4607, and
- the *Lactotoccus lactis spp lactis* 1 strain of lactic acid bacterium, strain NOL11, said strain being deposited at the CNCM under number CNCM I - 4609.

8. Composition for its use according to anyone of claims 1 to 7, wherein said farmed oviparous animals are birds and said composition is applied on the shell of the eggs of said birds.

9. Composition for its use according to anyone of claims 1 to 7, wherein said farmed oviparous animals are aquatic oviparous animals and said composition is dispersed in the water of the rearing tanks of said aquatic oviparous animals in order to be placed in contact with said eggs, or in order to be placed directly in contact with said eggs.

10. Composition for its use according to anyone of claims 1 to 9, wherein said infections of farmed oviparous animals occur in the first days after eggs eclosion.

11. Composition for its use according to anyone of claims 1 to 10, wherein said at least one strain of a bacterium of the genus Bacillus and said at least one strain of lactic acid bacterium are used simultaneously, separately or spread out over time.

12. Use of a composition comprising or consisting essentially of a mixture of:
a. at least one strain of a bacterium of the genus Bacillus, which is a strain of *Bacillus subtilis;* and
b. at least one strain of lactic acid bacterium, said strain of lactic acid bacterium being the *Lactotoccus lactis spp lactis 1* strain NOL11, said strain being deposited at CNCM under number CNCM I - 4609,
for improving zootechnical performances of farmed oviparous animals, said composition being placed in direct contact with the eggs of said farmed oviparous animals.

13. Use according to claim 12, in which the composition comprises:
- at least one of the following three strains of *Bacillus subtilis:* NOL01, NOL02, NOL03, said strains being deposited at the CNCM under respective numbers: CNCM I- 4606, CNCM 1-5043 and CNCM I - 4607, and
- at least the strain of lactic acid bacterium: *Lactococcus lactic spp lactis* 1 strain NOL11, said strain being deposited at the CNCM under number CNCM 1-4609.

14. Use according to claim 12 or 13, in which the composition comprises:
- the following three strains of *Bacillus subtilis:* NOL01, NOL02, NOL03, said strains being deposited at the CNCM under the respective numbers: CNCM I - 4606, CNCM 1-5043 and CNCM I - 4607, and
- the *Lactotoccus lactis spp lactis* 1 strain of lactic acid bacterium, strain NOL11, said strain being deposited at the CNCM under number CNCM I - 4609.
